# EUROPÄISCHE PATENTSCHRIFT

(11) **EP 4 164 493 B1**
(45) Veröffentlichungstag und Bekanntmachung des Hinweises auf die Patenterteilung: **22.05.2024**
(21) Anmeldenummer: 21733060.4
(22) Anmeldetag: 10.06.2021
(51) Int. Cl.: A61B 5/251, A61B 5/259, A61B 5/291

(54) **ELEKTRODE ZUR NICHTINVASIVEN MESSUNG VON ELEKTRISCHEN KÖRPERSIGNALEN AUF EINER HAUTOBERFLÄCHE**
ELECTRODE FOR THE NON-INVASIVE MEASUREMENT OF ELECTRIC BODY SIGNALS ON A SKIN SURFACE
ÉLECTRODE POUR LA MESURE NON INVASIVE DE SIGNAUX ÉLECTRIQUES CORPORELS SUR UNE SURFACE CUTANÉE

(30) Priorität: 15.06.2020 DE 102020115695
(43) Veröffentlichungstag der Anmeldung: 19.04.2023
(73) Patentinhaber: Karlsruher Institut für Technologie, 76131 Karlsruhe (DE)
(72) Erfinder: STOCK, Simon, 76135 Karlsruhe (DE); GERDES, Marius, 76131 Karlsruhe (DE); MAIER, Heiko, 76131 Karlsruhe (DE); KOVÁCS, Bálint, 69126 Heidelberg (DE); STORK, Wilhelm, 76829 Landau i. d. Pfalz (DE)
(86) Internationale Anmeldenummer: PCT/EP2021/025207
(87) Internationale Veröffentlichungsnummer: WO 2021/254659

(56) Entgegenhaltungen:
- JP-A- 2019 072 309
- US-A1- 2016 022 165

## Beschreibung

Die Erfindung betrifft eine Elektrode zur nichtinvasiven Messung von elektrischen Körpersignalen auf einer Hautoberfläche gemäß des ersten Patentanspruchs.

Eingangs genannte Elektroden sind Messaufnehmer für die Erfassung oder Eintragung von elektrischen Signalen auf einer Haut oder einer anderen Oberfläche eines Patienten oder eines anderen Lebewesens. Sie werden hierzu auf die Haut oder Oberfläche direkt aufgesetzt und stehen mit dieser in direktem elektrischen Kontakt. Entwickelt wurden sie für Diagnostik- und Therapieverfahren, beispielsweise für die Elektrokardiographie (EKG), Elektroenzephalografie (EEG), Elektromyografie (EMG) oder andere elektroneurologische Untersuchungen, aber auch beispielsweise für die Muskel- oder Nervenstimulation. Dabei ist es erforderlich, einen zuverlässigen und reproduzierbaren elektrischen Kontakt zwischen der Haut oder Oberfläche mit der Elektrode herzustellen und möglichst ohne Schwankungen des Widerstands zu halten. Üblicherweise kommt für diesen Zweck ein Ankopplungsmedium zur elektrischen Anbindung der Elektrode an die Messstelle auf der Haut oder auf der Oberfläche zum Einsatz.

Bei der genannten Anbindung der Elektrode an die Messstelle treten jedoch immer wieder Unregelmäßigkeiten auf, wenn die Messstelle selbst Unregelmäßigkeiten aufweist, hervorgerufen durch die Oberflächentopographie, Hautfalten, Verschmutzung, Wunden oder Behaarungen. Insbesondere Kopfhaare erschweren einen zuverlässigen Kontakt der Elektroden auf der Hautoberfläche.

Um dennoch eine zuverlässige Anbindung einer Elektrode auf einer Haut oder einer Oberfläche mit Unregelmäßigkeiten sicherzustellen, gibt es im Stand der Technik verschiedene Ansätze.

Üblicherweise wird eine zuverlässige elektrische Anbindung in praxi mit einer erhöhten Menge an Ankopplungsmedium angestrebt, was wiederum zu einer erhöhten Menge an Rückständen und damit ungewollte Verunreinigungen auf der Haut oder Oberfläche führt.

Aus der DE 10 2011 101 583 A1 ist beispielsweise eine Elektrode zum transkutanen Übertragen elektrischer Signale bekannt, bei der zwischen Elektrode und vorgenannter Messstelle eine Schicht vorgesehen ist, die geeignet ist, eine Flüssigkeit als Ankopplungsmedium kapillar festzuhalten und somit einen Austritt dieser zwischen Haut und Elektrode zu verhindern.

WO 2012/140629 A1 repräsentiert beispielhaft eine Elektrode mit einer Saugnapfanbindung.

Eine Elektrode für die Erfassung von Hirnströmen, die sich aus mehreren auf der Kopfhaut aufliegenden Elektrodenfüßen addiert, findet sich beispielhaft in WO 2014/152806 A1**.** Jeder der Elektrodenfüße weist hierzu einen Kanal auf, der ein Reservoir für ein Ankopplungsfluid und eine Öffnung am distalen Ende, d.h. an der auf der Kopfhaut auffliegenden Auflagefläche des Elektrodenfußes miteinander verbindet. Durch ein Nachfluss von Ankopplungsfluid aus dem Reservoir werden mögliche Leckageverluste im Bereich der Auflagefläche ausgeglichen.

Alle diese Ansätze ermöglichen jedoch keinen Austausch an Ankopplungsmedium während einer Messung, es sei denn, man nimmt die in der WO 2014/152806 A1 genannten Leckageverluste im größerem Umfang in Kauf. Eine Eliminierung von Störgrößeneinflüssen wie z.B. von Haaren hervorgerufen sind während der Messung folglich nur eingeschränkt möglich, was insbesondere die Durchführbarkeit von Langzeitmessungen einschränkt.

Auch aus der JP 2019-72 309 A eine Elektrode mit mehreren Elektrodenfüßen mit jeweils einer Auflagefläche bekannt. Wie in der WO 2014/152806 A1 weist auch diese Elektrode in den Elektrodenfüßen einen Kanal auf, der ein Reservoir für ein Ankopplungsfluid und eine Öffnung am distalen Ende abseits der Auflagefläche miteinander verbindet. Aus dieser Öffnung tritt das Ankopplungsfluid aus den Elektrodenfuß hervor und benetzt damit auch die Haut unter der Auflagefläche. Zur Absaugung des über die Öffnung austretenden Ankopplungsfluid weist jeder Elektrodenfuß zudem ebenfalls neben der Auflagefläche jeweils eine zweite Öffnung, eine Absaugöffnung für das zuvor ausgetretene Ankopplungsfluid auf, womit versucht wird, die Verluste an dem ausgetretenen Ankopplungsfluid zu reduzieren.

Davon ausgehend liegt eine **Aufgabe der Erfindung** darin, eine Elektrode so zu gestalten, dass diese einfacher handhabbar ist und insbesondere eine Durchführung auch von Langzeitmessungen auf einer dichtbehaarten Haut wie z.B. auf dem Kopf ohne die genannten Unregelmäßigkeiten ermöglicht.

Die Aufgabe wird mit einer Elektrode mit den Merkmalen des ersten Patentanspruchs gelöst. Die auf diese rückbezogenen Unteransprüche geben vorteilhafte Ausführungsformen wieder.

Die Lösung der Aufgabe basiert auf einer Elektrode zur nichtinvasiven Messung von elektrischen Körpersignalen auf einer Hautoberfläche. Die Elektrode umfasst ein Gehäuse mit einer ersten und einer zweiten Kavität für eine Elektrolytlösung als Ankopplungsmedium im Gehäuse, Mittel für ein Einbringen von Elektrolytlösung die beiden Kaviäten sowie mindestens eine Elektrolytelektrode, vorzugsweise eine korrosionsbeständige elektrisch leitfähige oder mit einer elektrisch leitfähigen Schicht versehene Elektrode, in einem der zwei Kavitäten. Elektrolytelektrode und/oder Schicht sind weiter bevorzugt aus Silber, Gold, Platin, Edelstahl (Chrom-Nickel-Stahl) oder einem anderen Metall oder einer anderen Metalllegierung.

Am Gehäuse ist mindestens ein, vorzugsweise mindestens drei Gehäusefüße mit je einer ringförmigen Auflagefläche für die Hautoberfläche am jeweiligen distalen Ende eines jeden Gehäusefußes vorgesehen.

Das Gehäuse ist entweder einstückig gestaltet oder aus mehreren Gehäusekomponenten zusammengesetzt.

Eine erste bevorzugte Ausgestaltung sieht ein Gehäuse mit mehreren Komponenten, d.h. einem nach oben hin offenes Gehäuse als erste Gehäusekomponente mit separatem Deckel zum Verschließen des offenen Gehäuses als zweite Gehäusekomponente vor. Mit der Abnahme des Deckels vom offenen Gehäuse werden mindestens eine der beiden Kavitäten zugänglich.

In der vorgenannten ersten bevorzugten Ausgestaltung des Gehäuses mit mehreren Gehäusekomponenten besteht jenes aus einer ersten Gehäusekomponente mit dem Gehäusefuß oder den Gehäusefü-ßen und den Kavitäten sowie einer zweiten Gehäusekomponente, einem Deckel, in der die Elektrolytelektrode eingesetzt ist. Die Kavitäten, in denen die Elektrolytelektrode eingesetzt ist, sind dabei in der ersten Gehäusekomponente offen gestaltet und werden durch die als Deckel dienende zweite Gehäusekomponente abgedeckt. Das Gehäuse, vorzugweise die bevorzugte Ausgestaltung mit den vorgenannten Gehäusekomponenten sind vorzugsweise aus einem Kunststoff, weiter bevorzugt hergestellt mittels einem additiven Fertigungsverfahren.

Alternativ ist auch eine Ausgestaltung des Deckels zugleich als Elektrolytelektrode denkbar, wobei der Deckel dann aus einem elektrisch leitfähigen Material besteht oder zumindest zur abgedeckten Kavität mit einem solchen beschichtet ist.

Eine zweite bevorzugte Ausgestaltung sieht ein einteiliges Gehäuse ohne separatem Deckel auf. Elektrolytelektroden werden vorzugsweise nachträglich über Schlitze im Gehäuse eingeschoben, wobei jeder Schlitz und damit jede eingeschobene Elektrolytelektrode an zumindest eine der beiden Kavitäten angrenzt.

Das Gehäuse selbst ist vorzugweise aus einem elektrisch nicht leitfähigen Material, wobei eine Elektrolytelektrode der vorgenannten Art vorgesehen sein muss.

Alternativ ist anstelle einer separaten Elektrolytelektrode auch das Gehäuse oder Gehäusekomponenten aus einem elektrisch leitfähigen Material herstellbar und als Elektrolytelektrode verwendbar.

Eine Ausführungsform mit nur einem Gehäusefuß bietet sich insbesondere bei flachen Bauformen der Elektrode mit vergleichsweise großer Auflagerfläche an, vorzugsweise dann, wenn die lateralen Abmessungen der Auflagefläche zumindest die halbe Höhe des Gehäuses übersteigt und damit die Kippgefahr auf einer Hautoberfläche gegenüber höheren Bauformen und/oder Bauformen mit kleineren Auflagefläche reduziert.

Eine Ausführungsform mit mindestens drei Gehäusefüßen ermöglicht in vorteilhafter Weise eine Applizierung der Elektrode über mehrere zueinander beabstandeten Auflageflächen auf einer Hautoberfläche, was wiederum eine erhöhte Kippsicherheit der Elektrode auch bei in Summe kleiner Auflagefläche insgesamt bewirkt.

Der Gehäusefuß oder die Gehäusefüße sind vorzugsweise Extremitäten des Gehäuses, die auf einer Seite des Gehäuses abstehen und somit in ihrer Gesamtzahl über ihre Auflageflächen zugleich auf die Hautoberfläche aufsetzbar und applizierbar sind. Die auf die Hautoberfläche aufgesetzten ringförmigen Auflageflächen umgeben dabei jeweils einen Bereich der Hautoberfläche, d.h. die eigentliche Messstelle. Ringförmig heißt im Rahmen der Lösung nicht zwingend, sondern nur vorzugsweise kreisförmig rund. Ringförmig ist im Sinne einer linienförmig umlaufenden Auflagefläche mit oder ohne Ecken zu verstehen. Es umfasst auch Ausgestaltungen mit Einbuchtungen oder auch andere Formen, bei denen die Krümmungsrichtung wechselt. Die Auflageflächen befinden sich am jeweils distalen Ende der Gehäusefüße. Vorzugsweise weist ein Gehäusefuß nur eine ringförmige Auflagefläche auf.

Jeder Gehäusefuß wird von zumindest, vorzugsweise genau zwei Kanälen durchdrungen, zumindest je einem Kanal vom distalen Ende zur ersten und zur zweiten Kavität. Beide Kanäle sind zum distalen Ende hin offen und werden im Gehäusefuß am distalen Ende von der ringförmigen Auflagefläche umgeben in ein Umlenkungsvolumen zusammengeführt. Liegen die Gehäusefüße über ihre Auflageflächen auf einer Haut oder einer anderen Oberfläche auf, wird das Umlenkvolumen durch die Haut nach außen hin verschlossen.

Vorzugsweise sind der Gehäusefuß oder die Gehäusefüße aus einem elastischen Material, vorzugsweise aus Kunststoff oder einem Elastomer hergestellt, zumindest aber der Bereich um die Auflagefläche kippelastisch nachgiebig ausgestaltet, sodass sie geeignet sind, sich im Bereich der Auflagefläche an eventuelle Unebenheiten auf der Haut oder Oberfläche besser anzugleichen. Dies erhöht in vorteilhafter Weise die Dichtigkeit im Bereich der Auflagefläche und reduziert weiter die Wahrscheinlichkeit von Leckageverlusten durch eventuell austretende Elektrolytlösung. Weiter bevorzugt ist das Material des Gehäuses einschließlich der Gehäusefüße fluiddicht gegenüber der Elektrolytlösung.

Eine bevorzugte Ausgestaltung der Auflagefläche der Elektrode sieht vor, diese als Dichtringe zu der Hautoberfläche auszugestalten, entweder als separate Dichtringe oder als angeformte Dichtringe, vorzugsweise jeweils mit einer Dichtlippe am distalen Ende der Gehäusefüße. Diese Dichtringe und/oder die Dichtlippen sind vorzugsweise aufgrund ihres Materials (vorzugsweise Gummi oder einem anderen Elastomer) und/oder ihrer Gestaltung (z.B. dünnwandige Dichtlippe und/oder hohler Dichtring) elastisch nachgiebiger als die Gehäusefüße.

Für die Elektrolytlösung besteht über die beiden Kanäle und dem verschlossenen Umlenkvolumen eine fluidische Verbindung zwischen den beiden Kavitäten, wobei die Elektrolytlösung den von der ringförmigen Auflagefläche umgebenden Bereich der Haut, d.h. die Messstelle, kontaktiert. Die Elektrolytlösung weist somit einen direkten Kontakt zur Messstelle einerseits und zu der mindestens einen Elektrolytelektrode in einem der zwei Kavitäten auf. Die jeweils beiden Kanäle der Gehäusefüße, d.h. die fluidischen Verbindungen durch die Gehäusefüße zwischen den beiden Kavitäten sind vorzugsweise parallel zueinander angeordnet.

Eine bevorzugte Ausgestaltung der Elektrode sieht vor, die distalen Enden, weiter bevorzugt auch der ringförmigen Auflageflächen auf eine gemeinsamen Ebene aufzuspannen, womit eine sichere Applikation auf ebenen oder nur gering gekrümmten Oberflächen begünstigt wird.

Weiter bevorzugt sind alle distalen Enden kreisförmig zueinander angeordnet. Damit wird in vorteilhafter Weise eine sichere Auflage der Auflageflächen aller Gehäusefüße insbesondere bei einer gewölbten Oberfläche, wie z.B. einer Kopfhaut ermöglicht.

Ein auf die Haut oder eine Oberfläche aufgesetzter Gehäusefuß liegt vorzugsweise mit seiner gesamten Auflagefläche auf der Haut. Ein Elektrolytlösungsstrom führt durch die Kanäle an dem von der ringförmigen Auflagefläche umgebenden Bereich der Haut, d.h. der Messstelle, vorbei und strömt ihn an. Leckageverluste an der Kontaktstelle zwischen der Auflagefläche und der Haut oder Oberfläche, hervorgerufen durch eine Oberflächentopographie, Hautfalten, Verschmutzung, Wunden oder Behaarung werden durch den Elektrolytlösungsstrom, der vorzugsweise in einem geschlossenen Kreislauf an der Messstelle vorbeigeführt wird, und/oder mit einer erhöhten Menge an Ankopplungsmedium ausgeglichen. Ferner dient der genannte Elektrolytlösungsstrom einem Abtransport von ungewollten Verunreinigungen auf der Haut oder Oberfläche. Insbesondere diese Verunreinigungen, aber auch die obersten Hautschichten weisen einen erhöhten elektrischen Widerstand auf, was wiederum die Messung erschwert.

Eine weitere bevorzugte Ausführung kennzeichnet sich dadurch aus, dass die erste und die zweite Kavität sowie die dazwischen angeordneten Kanäle in den Gehäusefuß oder Gehäusefüßen eine Fluidleitung für die Elektrolytlösung bilden und die Fluidleitung ein Teil eines Elektrolytkreislaufs ist. Der Elektrolytkreislauf bewirkt in vorteilhafter Weise eine laufende Anströmung der vorgenannten Messstellen mit Elektrolytlösung und damit einen permanenten Abtransport von Verunreinigungen von der Messstelle.

Eine weitere bevorzugte Ausführung kennzeichnet sich dadurch aus, dass ein Zulauf für eine Elektrolytlösung zu der ersten Kavität im Gehäuse sowie einen Ablauf für die Elektrolytlösung aus der zweiten Kavität im Gehäuse für eine Elektrolytlösung vorgesehen ist. Vorzugsweise aber nicht zwingend sind die Kavitäten, die vorgenannten Kanäle sowie den Zu- und Ablauf Teil eines Elektrolytlösungskreislaufes, wobei die Stromrichtung zunächst von der ersten Kavität durch die Kanäle und das Umlenkvolumen in die zweite Kavität erfolgt. Vorzugsweise findet die Umwälzung des Elektrolytlösungskreislaufes durch Umwälzmittel außerhalb des Gehäuses statt.

Im Umlenkvolumen erfolgt die Aufnahme von möglichen Verunreinigungen und damit eine Kontamination der Elektrolytlösung. Daher wird im Rahmen einer Ausgestaltung vorgeschlagen, die Elektrolytelektrode in der ersten Kavität anzuordnen, sodass die Übertragung der elektrischen Signale von der Messstelle zur Elektrolytelektrode stets in einem Strömungsbereich vor der Kontamination stattfindet. Störungen durch die Kontamination werden damit ausgeschlossen.

Eine alternative Ausgestaltung weist anstelle des Elektrolytlösungskreislaufs eine in Strömungsrichtung wechselnde zyklische Strömung auf. Die Elektrolytlösung wird dabei vorzugsweise durch die Kanäle und das Umlenkungsvolumen zwischen den beiden Kavitäten hin- und hergeleitet, getrieben vorzugsweise durch einen Pulserzeuger, beispielsweise eine Membranpumpe (z.B. extern oder in der ersten Kavität mittels mechanisch angesteuerter Membran, die vorzugsweise auch als Elektrolytelektrode nutzbar ist), die hydraulisch gegen ein elastisches Element (z.B. Luftblase in der zweiten Kavität) wirkt. Der laufende, vorzugsweise zyklische Richtungswechsel der Strömung im Umlenkungsvolumen bewirkt in besonders vorteilhafter Weise eine erhöhte Ablösung von Verunreinigungen in der Messstelle.

Die Erfindung wird anhand von weiteren Ausführungsbeispielen, den folgenden Figuren und Beschreibungen näher erläutert. Alle dargestellten Merkmale und deren Kombinationen sind nicht nur auf diese Ausführungsbeispiele und deren Ausgestaltungen begrenzt. Vielmehr sollen diese stellvertretend für weitere mögliche, aber nicht explizit als Ausführungsbeispiele dargestellte weitere Ausgestaltungen kombinierbar angesehen werden. Es zeigen
**Fig.1** eine Schnittdarstellung einer beispielhaften Elektrode gemäß des Stands der Technik (Kanalelektrode mit einem Kanal pro Gehäusefuß),
**Fig.2a** **bis d** eine Ansicht, zwei Schnittdarstellungen und eine Draufsicht einer Ausführungsform einer Elektrode mit mehreren Gehäusekomponenten und sieben Gehäusefüßen,
**Fig.3** eine graphische Darstellung von vergleichende Messungen an einer herkömmlichen Trockenelektrode ohne und mit einem Ankopplungsmedium sowie mit einer in **Fig.2a** **bis d** dargestellten Ausführungsform (Stimulationsfrequenz und deren harmonischen Oberfrequenzen des Stimulus über die Anzahl der Probennahmen),
**Fig.4** eine Ansicht einer beispielhaften Anordnung von mehreren Elektroden auf einer Kopfhaut mit Mitteln einer seriellen Versorgung der Elektroden mit Elektrolytlösung in einem geschlossenen Elektrolytkreislauf,
**Fig.5a** **und b** je eine Prinzipskizze weiterer Ausführungsformen einer Elektrode mit in dieser eingesetzten und auf die Messstelle auf der Auflagefläche einwirkenden Ultraschallquellen sowie einer eingesetzten Elektrolytelektrode (a) bzw. einer durch ein elektrisch leitfähiges Material des Gehäuses gebildete Elektrolytelektrode (b),
**Fig.6a** **bis c** eine Ansicht sowie zwei Schnittdarstellungen einer weiteren Ausführungsform einer Elektrode mit nur einer Gehäusekomponente, eingeschobener Elektrolytelektrode und drei Gehäusefüßen,
**Fig.7** eine Schnittdarstellung einer weiteren Ausführungsform einer Elektrode mit einer eingeschobenen Elektrolytelektrode sowie einem integralen auf die Elektrolytlösung einwirkenden Temperierungsmittel,
**Fig.8** eine Ansicht einer Brille als Elektrodenhalterung mit Aufnahmen für Elektroden sowie
**Fig.9** eine Ansicht einer Videobrille mit Aufnahmen für Elektroden.

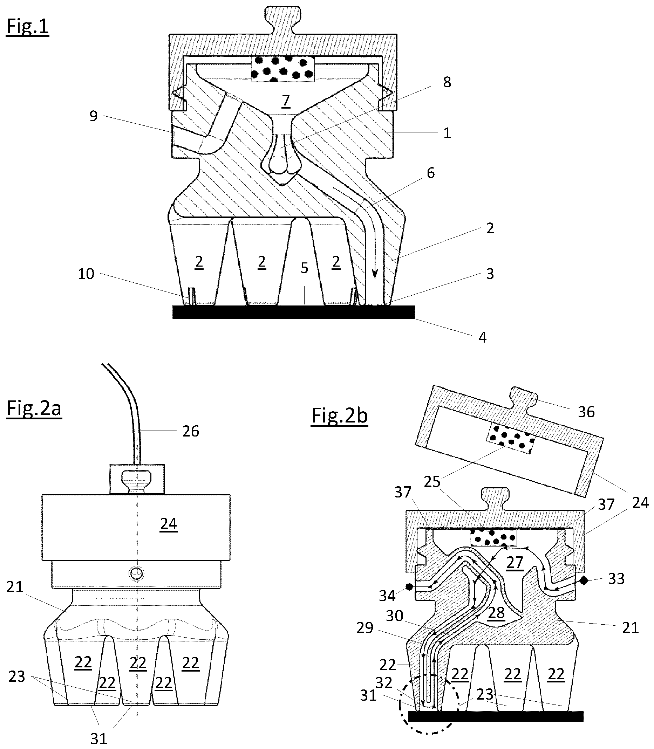

Die Ausführungsform gemäß **Fig.1** repräsentiert einen Stand der Technik einer Elektrodenbauform mit Gehäuse **1** und Gehäusefüßen **2,** deren distalen Enden **3** Auflageflächen **4** zu einer Hautoberfläche **5** bilden. In jedem Gehäusefuß befindet sich ein Kanal **6,** der von einer Kavität **7** mit Zulauf **9** im Gehäuse über einen Verteilerkanalabschnitt **8** zum jeweiligen distalen Ende führt und dort ausmündet. Die Auflageflächen **4** sind vorzugsweise ringförmig eben, unterbrochen nur von Einstichen **10** in das distale Ende **3** eines jeden Gehäusefußes. Ferner sind in **Fig.1** noch eine Elektrolytelektrode **11** unter einem Deckel **12** zur Abdeckung der Kavität 7 dargestellt.

Die Elektrolytlösung wird von dem Zulauf in die Kavität 7 und von dort über die Kanäle **6** zu den distalen Enden geführt, wo sie aus den Einstichen **10** austritt. Nicht in **Fig.1** dargestellt sind eine Zuführungsleitung zum Zulauf.

**Fig.2a** **bis d** repräsentieren dagegen ein Ausführungsbeispiel der beanspruchten Elektrode mit Gehäuse **21,** Gehäusefüßen **22** mit distalen Enden **23** und ringförmigen Auflageflächen **31** sowie einen Deckel **24** mit einer in diesem angeordneten Elektrolytelektrode **25** mit Messkabel **26.** Die ringförmigen, bevorzugt die dargestellten kreisringförmigen Auflageflächen umschließen jeweils eine Messstelle **35** auf der Hautoberfläche **5.** Das Gehäuse ist vorzugsweise aus einem elektrisch nicht leitfähigen Material, vorzugsweise Kunststoff gefertigt, weiter bevorzugt mittels einem additiven Fertigungsverfahren hergestellt. Als additive Fertigungsverfahren für die Herstellung der Elektrode eigneten sich in ersten erfolgreichen Versuchen SLA-Verfahren mit einem Resin oder andere 3D-Druckverfahren mit Strukturauflösung vorzugsweise zwischen 0,2 und 0,5 mm.

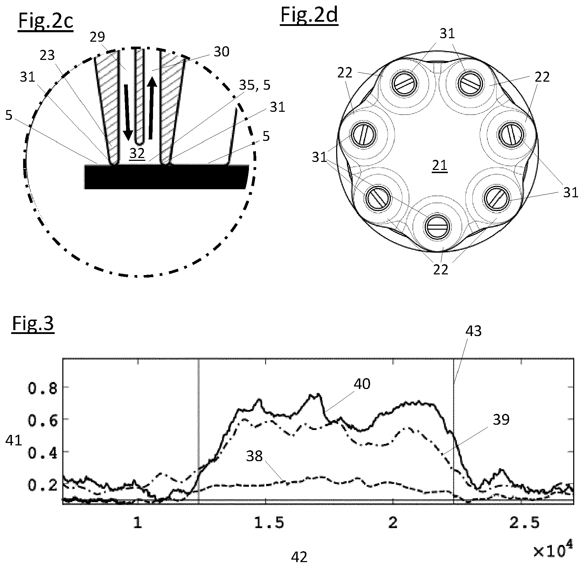

Im Gegensatz zu dem in **Fig.1** dargestellten Stand der Technik weist das Ausführungsbeispiel anstelle nur einer Kavität zwei Kavitäten, eine erste Kavität **27** und eine zweite Kavität **28,** auf. Ferner zeichnet sich das Ausführungsbeispiel durch zwei Kanäle je Gehäusefuß aus, je einen ersten Kanal **29** von der ersten Kavität **27** zum distalen Ende eines jeden Gehäusefußes **22** und einen zweiten Kanal **30** von dem distalen Ende eines jeden Gehäusefußes **22** zur zweiten Kavität **28 (vgl.** **Fig.2b****).** Beide Kanäle sind zum distalen Ende hin offen und werden im Gehäusefuß vor dem distalen Ende von der ringförmigen Auflagefläche **31** umgeben in ein Umlenkungsvolumen **32** zusammengeführt. **Fig.2c** gibt Bereich um das Umlenkvolumen eines auf der Hautoberfläche **5** aufgesetzten distalen Ende eines Gehäusefußes **23** als Ausschnittsvergrößerung aus **Fig.2b** wieder. Im Umlenkvolumen erfolgt eine Umlenkung der der Elektrolytlösungsströmung vom ersten in den zweiten Kanal, wobei mögliche Verunreinigungen wie Hautschuppen, Schmutz, aber auch Haare von der Strömung aufgenommen über den zweiten Kanal abtransportiert werden. Folglich ist die Elektrolytlösung im ersten Kanal noch nicht verunreinigt und damit in seinen elektrischen Eigenschaften grundsätzlich nicht verändert. Eine Anordnung der Elektrolytelektrode im Elektrolytstrom vor der Verunreinigung wird angestrebt, um die Einflüsse durch jene weitestgehend auszuschließen. Folglich ist die Elektrolytelektrode im Ausführungsbeispiel vorzugsweise zentral in der ersten Kavität angeordnet. Die von der Elektrolytlösung zu überbrückende Entfernungen zwischen Elektrolytlösungselektrode zu den Messstellen **35** sind für jeden Elektrodenfuß vorzugsweise jeweils gleich, weiter bevorzug auch im Querschnitt der Elektrolytströmung jeweils gleich.

Im Gegensatz zu **Fig.1** zeigen **Fig.2a** **bis d** keine Unterbrechung der ringförmig umlaufenden Auflageflächen **31** durch in **Fig.1** beispielhaft dargestellte Einstiche (vgl. insbes. **Fig.2d****).** Ein Austritt von Elektrolytlösung aus einem Einstich nach außen ist mangels jenem folglich nicht möglich. Liegt eine Auflagefläche **31** - wie in **Fig.2b** **und c** dargestellt - über dessen gesamte ringförmige Erstreckung auf einer Hautoberfläche **5** auf, so ist das Umlenkungsvolumen **32** durch diesen Kontakt zwischen (umlaufender) Auflagefläche und Hautoberfläche folglich nach außen hin abgeschlossen.

Die erste Kavität **27** weist vorzugsweise einen Zulauf **33,** die zweite Kavität **28** vorzugsweise einen Ablauf **34** von bzw. zu einer nicht weiter dargestellten Elektrolytquelle bzw. -senke auf. Die Strömungsrichtung der Elektrolytlösung in der Elektrode wird durch Pfeile in **Fig.2b** **und c** wiedergegeben.

In der Schnittdarstellung **Fig.2b** ist der Deckel **24** in zwei Positionen dargestellt, einmal aufgesetzt auf dem Gehäuse **21** direkt über der ersten Kavität **27** und einmal darüber in abgehobener Position. Unter den Deckel ist die Elektrolytelektrode **25** befestigt, die in der optional dargestellten aufgesetzten Position in die erste Kavität **27** in die darin befindliche Elektrolytlösung hineinragt. Der Deckel selbst ist vorzugsweise aus einem elektrisch nicht leitfähigen Material gefertigt, vorzugsweise einem Kunststoff. Die Fixierung auf dem Gehäuse erfolgt vorzugsweise mittels eines Schraubgewindes, wobei eine Deckeldichtfläche **37** einen Austritt von Elektrolytlösung aus der ersten Kavität verhindert. Alternativ lässt sich der Deckel auch mit einer umlaufenden Schnapp- oder Einrastverbindung auf dem Gehäuse fixieren, wobei der Deckel dann bevorzugt aus einem Elastomer oder Gummi gefertigt ist.

Der Deckel weist im dargestellten Ausführungsbeispiel ferner einen Anschlussnippel **36** mit einer nicht weiter dargestellten Durchkontaktierung zur Elektrolytelektrode für das Messkabel **26** auf. Der Anschlussnippel ist Teil einer in vorteilhafter Weise verdrehungsfähigen Druckknopfverbindung für eine messkabelseitige Druckknopfaufnahme, schematisch dargestellt in **Fig.2a****.**

**Fig.2d** gibt die gehäusefußseitige Draufsicht des dargestellten Ausführungsbeispiels wieder. Die Elektrode weist beispielhaft sieben Gehäusefüße **22** mit kreisringförmigen Auflageflächen **31** auf, die wie dargestellt vorzugsweise im Kreis und weiter bevorzugt in regelmäßigen Abständen zueinander angeordnet sind.

**Fig.3** zeigt vergleichende Messungen **38, 39, 40** an einer herkömmlichen Trockenelektrode ohne (trockene Elektrode, **(38))** und mit einem Ankopplungsmedium (Ankopplungsgel, Gelelektrode, **(39))** sowie mit einer in **Fig.2a** **bis d** dargestellten Ausführungsform **(40).** Aufgetragen ist die Stimulationsfrequenz und deren harmonischen Oberfrequenzen **41** des Stimulus (dimensionslos, angepasst mittels einer Kanonische Korrelationsanalyse in einem EEG-Signal über die Anzahl **42** der Probennahmen N in [x10⁴]. Ein Stimulus wurde ab der gelben Maskierung präsentiert. Dieser währte bis zur zweiten Markierung **43.** Ein höherer Wert während des Stimulus deutet auf eine bessere Erfassbarkeit hin. Gemessen wurde bei ein und derselben Person zu gleicher Zeit und an vergleichbaren Messstellen. In jeden Fall ist das Signal zu Rausch-Verhalten sowohl bei einer Lidschlagerkennung als auch bei visuellen Stimuli besser als bei trockenen Elektroden und gleichwertig mit Gelelektroden.

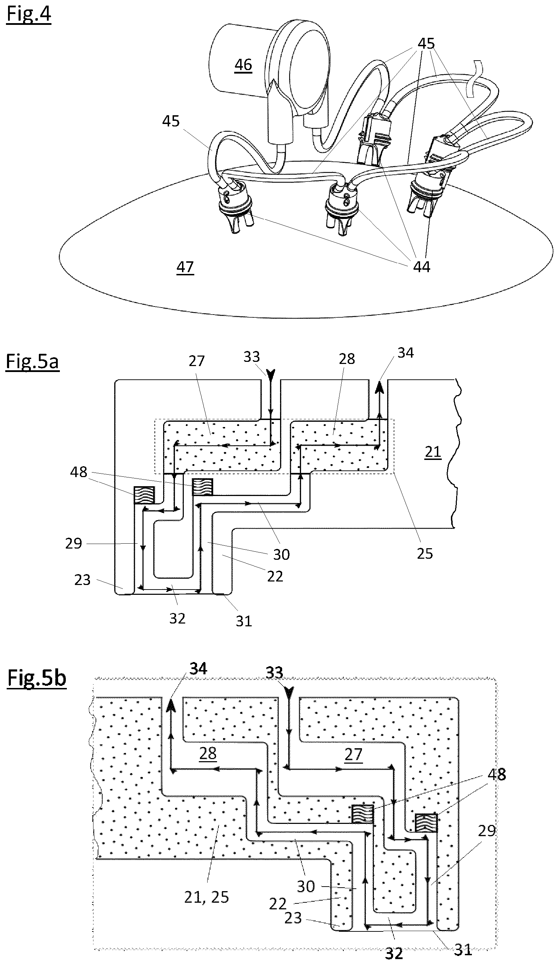

**Fig.4** zeigt eine Ansicht einer beispielhaften Anordnung von mehreren Elektroden **44** auf einer Kopfhaut **47** mit Mitteln einer seriellen Versorgung der Elektroden mit Elektrolytlösung in einem geschlossenen Elektrolytkreislauf. In der dargestellten Form umfassen diese Mittel eine Umwälzpumpe **46,** (vorzugsweise mit Fluidreservoir und Filterelementen für den Elektrolytkreislauf), mit der mittels einem geschlossenen Elektrolytlösungskreislauf die Elektroden über Schlauchleitungen **45** seriell mit einer Elektrolytlösung durchströmt werden.

Prinzipskizzen von zwei weiteren Ausführungsbeispielen einer Elektrode mit im Gehäuse **21** eingesetzten und auf die Messstelle auf der Auflagefläche einwirkenden Ultraschallquellen **48,** vorzugsweise piezoelektrische piezokeramische Wandlerelemente mit einer Ummantelung mit Auskopplungsschicht aus einem elektrisch isolierenden Material zur Elektrolytlösung hin, geben **Fig.5a** **und b** wieder. Dargestellt ist lediglich ein Teil des Gehäuses und ein Gehäusefuß ohne Hautoberfläche, aber mit Pfeilen dargestellt die Fließrichtung des Elektrolytkreislaufs. Die Messstellen sind folglich nicht weiter dargestellt, werden aber im applizierten Betriebszustand von der Auflagefläche **21** am distalen Ende **23** des Gehäusefußes **22** begrenzt. Die Ultraschallquellen sind in den ersten und zweiten Kanälen **29** und **30** angeordnet und in diesen in Richtung des Umlenkvolumens **32** und damit direkt auf den von der ringförmigen Auflagefläche **31** umschlossenen Messstelle auf der Hautoberfläche gerichtet. Durch eine Einwirkung von Ultraschallenergie lösen sich Haut- und Schmutzbestandteile in der Messstelle, reduzieren damit den elektrischen Kontaktwiderstand und tragen so maßgeblich zu einer besseren Anbindung der Elektroden bei. Beide Ausführungsformen weisen Elektrolytelektrodenflächen auf, die beispielhaft auch für andere Ausführungsformen vor und nach dem Umlenkbereich **32** mit dem Elektrolytkreislauf im elektrischen Kontakt stehen und damit gegenüber einem Elektrolytkontakt nur vor oder nur nach dem Umlenkbereich eine weiter verbesserte elektrische Anbindung bewirken.

Eine nicht weiter dargestellte Ausführungsform mit auf die Auflagefläche einwirkenden Ultraschallquellen **48,** zeichnet sich dadurch aus, dass die Ultraschallquelle direkt über dem Umlenkvolumen **32** angeordnet sind und direkt durch jenes auf die Messstelle ausgerichtet ist. Vorzugsweise bildet das Gehäusematerial im Gehäusefuß zwischen den beiden Kanälen eine Trennwand, die teilweise oder ganz als piezoelektrischer Wandler oder als Auskopplungselement zwischen einem Ultraschallwandler und dem Umlenkvolumen gestaltet ist. Der Wert der akustischen Impedanz des Auskopplungselements liegt vorzugsweise mittig zwischen denen der Elektrolytlösung und dem des Ultraschallwandlers Vorzugsweise besteht das Auskopplungselement aus dem Gehäusematerial. Weiter bevorzugt ist das Auskopplungselement und das Gehäuse einstückig aus ein und demselben Material gebildet, wobei es zwischen Ultraschallwandler und Elektrolytlösung zu einer vollständigen elektrischen Trennung durch das Material kommt.

Die Auskopplungselemente sind vorzugsweise als Auskopplungsschichten integraler Bestandteil der Gehäusefüße und bilden weiter bevorzugt eine fluiddichte elektrisch isolierende Barriere zwischen den Wandlerelementen und den beiden Kavitäten, den Kanälen und dem Umlenkvolumen, d.h. die Elektrolytlösung und die Wandler (insbesondere die Wandlerelektroden) sind in vorteilhafter Weise elektrisch und fluidisch voneinander getrennt.

Anstelle der Ultraschallquellen lassen sich grundsätzlich auch andere Schall- oder Vibrationsquellen wie z.B. Vibrationsmotoren einsetzen, die geeignet sind, in die Elektrolylösung entweder Ultraschallwellen, Schallimpulse oder Drucksignale oder - signalfolgen einzutragen oder die Elektroden an der Kontaktstelle zur Hautoberfläche, d.h. die vorgenannten Auflageflächen, so in Vibration zu versetzen, sodass sich Hautschuppen oder Verunreinigungen ablösen. Durch an Reibung an den Kontaktstellen lassen sich insbesondere Hautschuppen entfernen, was in der herkömmlichen medizinischen Praxis mit Schmirgelpapier durchgeführt wird. Durch die vorgenannten Vibrationsquellen sind die Auflageflächen der Elektroden auf der Kopfhaut durch Vibration bewegbar und man bildet damit das Schmirgeln nach. Optional sind die Auflageflächen hierzu aufgerauht. Ferner wird optional vorgeschlagen, die Vibration nicht dauerhaft, sondern nur bei Bedarf einzusetzen, beispielsweise angetriggert durch eine nachlassende Signalqualität. Vibrationsmotoren für die Kontaktstellen sind preiswert und miniaturisiert aus der Handy Technologie erhältlich.

**Fig.5a** repräsentiert beispielhaft eine Ausführungsform, bei der eine (gestrichelt dargestellte) Elektrolytelektrode **25** im Gehäuse **21** integriert ist und im Beispiel (d.h. nicht zwingend) sowohl in der ersten als auch in der zweiten Kavität **27** bzw. **28** unmittelbar im elektrischen Kontakt zu dem Elektrolytkreislauf steht. Das Gehäuse selbst ist elektrisch nicht leitfähig, vorzugsweise aus einem Kunststoff, weiter bevorzugt mittels eines additiven Fertigungsverfahrens oder eines Spritzgussverfahrens hergestellt. Die Elektrolytelektrode ist entweder während der Gehäusefertigung als Einlegekomponente integriert oder in ein entsprechend vorgesehenes Einschubfach nachträglich eingeschoben worden.

**Fig.5b** repräsentiert beispielhaft eine weitere Ausführungsform, bei der das Gehäuse **21** selbst aus einem elektrisch leitfähigen Material bestehend die Elektrolytelektrode **25** für beide Kavitäten **27** und **28,** aber auch für die beiden Kanäle **29** und **30** bildet. Eine separate Elektrolytelektrode ist hier nicht vorgesehen. Das elektrisch leitfähige Material ist ein gegenüber dem Elektrolyten korrosionsbeständigen Material, wie es auch zuvor für die separaten Elektrolytelektroden vorgeschlagen wird. Die Fertigung des Gehäuses erfolgt vorzugsweise durch ein additives Fertigungsverfahren (z.B. Lasersintern) oder alternativ durch ein Druckgussverfahren, Sinterverfahren oder durch galvanische Abscheidung, wobei die Kavitäten, z.B. durch vor der Herstellung eingesetzte nachträglich thermisch oder chemisch entfernbare Kerne realisierbar sind.

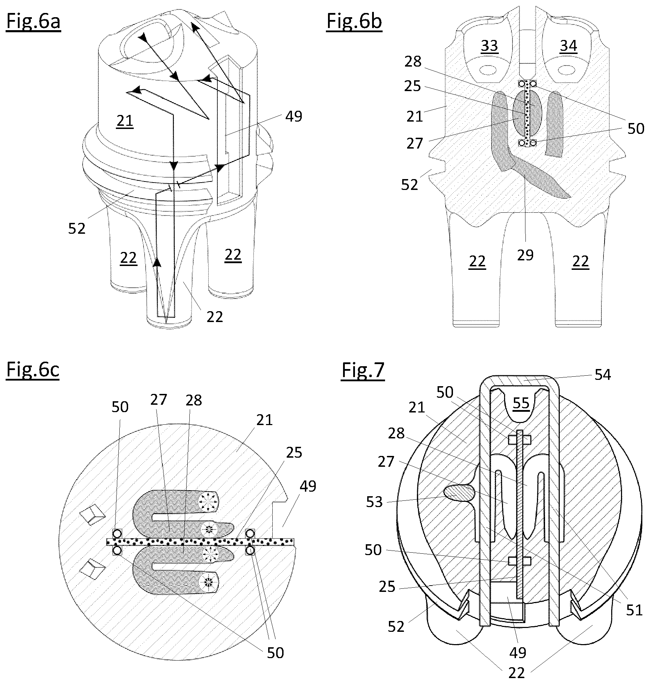

**Fig.6a** **bis c** zeigen beispielhaft eine Ansicht sowie zwei Schnittdarstellungen einer weiteren Ausführungsform einer Elektrode mit nur einer Gehäusekomponente, einer über einen Einschub **49** in das Gehäuse **21** eingeschobener Elektrolytelektrode **25** und drei Gehäusefüßen **22.** In **Fig.6a** ist zudem die Fließrichtung des Fluidkreislaufs schematisch durch Pfeile dargestellt, wobei die Fließrichtung beidseitig der Elektrolytelektrode verläuft. Die Elektrolytelektrode wird durch ein Blechstreifen oder Balken gebildet und beidseitig vom Elektrolyten kontaktiert. Sie bildet zugleich die Trennung zwischen den beiden Kavitäten **27** und **28,** wobei Dichtungen **50** die Kavitäten gegeneinander und zum Einschub **49** hin fluiddicht abdichten. Der Zu- und Ablauf **33** bzw. **34** zu den Kavitäten ist vorzugsweise als genormter Schnellsteckanschluss gestaltet, was wie der für die Befestigung der Elektrode an eine ebenfalls vorzugsweise genormte Elektrodenhalterung vorgesehene Haltebund/Halterille **52** in vorteilhafter Weise eine schnelle Rüstzeit und Austauschbarkeit der Elektrode im medizintechnischen System begünstigt.

**Fig.6c** zeigt zudem in einer Schnittdraufsicht in den beiden Kavitäten **27** und **28** schematisch rund dargestellte Zuläufe (Pfeile darin radial nach außen gerichtet) und Ausläufe (Pfeile darin radial nach innen gerichtet) in bzw. aus den Kavitäten aus bzw. in die in dieser Figur nicht weiter dargestellten Kanäle **29** bzw. **30** sowie zum Zu- und Ablauf **33** bzw. **34.**

**Fig.7** zeigt eine Schnittdarstellung einer weiteren beispielhaften Ausführungsform einer Elektrode mit einer eingeschobenen Elektrolytelektrode **25.** Sie unterscheidet sich von der in den **Fig.6a** **bis c** dargestellten Ausführung insbesondere darin, dass Temperierungsmittel vorgesehen sind. Dies umfassen im Beispiel eine in das Gehäuse **21** in oder durch beide Kavitäten **27** und **28** eingeschobene Wärmeübertragungsstruktur **51,** die mit einem Ende **54** aus dem Gehäuse ragt und mit einer externen Temperierungsquelle thermisch beaufschlagbar ist. Für diese externe Temperierungsquelle, vorzugsweise ein Heizelement ist vorzugsweise im Gehäuse eine Einbuchtung **55** vorsehen. In diese wird die Temperierungsquelle eingesetzt, durch das U-förmige Ende fixiert und in vorteilhafter Weise nach außen hin gleich mit abgeschirmt. Die Temperierung wird laufend durch einen Temperaturmessfühler **53** gemessen, vorzugsweise im Elektrolytkreislauf in der ersten Kavität, d.h. vor einer Weiterleitung an die Messstelle.

Anstelle einer separaten Wärmeübertragungsstruktur **51** wird im Rahmen einer weiteren Ausgestaltung vorgeschlagen, die Elektrolytelektrode für eine Wärmeübertragung in die Temperierungsmittel einzubinden. Ebenso wird alternativ vorgeschlagen, die Wärmeübertragungsstruktur als Fluidleitungen für ein Temperierungsfluid auszugestalten.

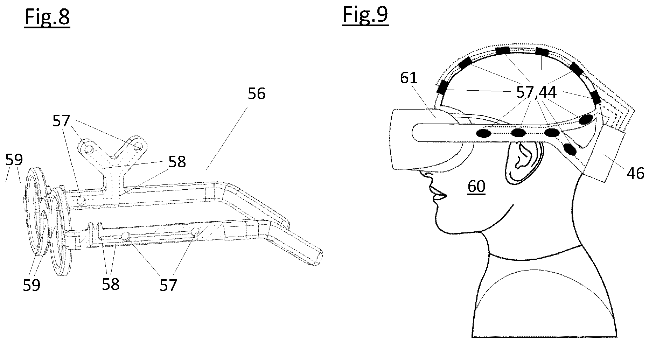

**Fig.8** zeigt eine Ansicht einer Brille **56** für einen Patienten als Elektrodenhalterung mit Aufnahmen **57** für Elektroden. Sie dient insbesondere der Erfassung von Hirnströmen, während der Patient visuellen Einflüssen ausgesetzt ist. Die Aufnahmen sind an festen Positionen im Brillengestell angeordnet und ermöglichen somit eine in vorteilhafter Weise eine (durch die Aufnahme der Nase und den Ohren vorgegebene) reproduzierbare Anordnung der Elektroden auf der Kopfhaut des Patienten und damit die Möglichkeit, die Messpositionen bei eventuellen Nachmessungen wieder vorzusehen. Die Aufnahmen sind vorzugsweise aus einem elastischen Material, vorzugsweise Gummieinsätzen mit den dargestellten Bohrungen, gebildet und ermöglichen somit insbesondere mit den in **Fig.6a** **und b sowie 7** dargestellten Haltebund/- rillen eine Einrastverbindung der Elektroden in der Brille. Schematisch dargestellt sind im Brillengestell optional integrierte Leitungen **58** zur Versorgung der Elektroden mit Elektrolytlösung. Über Nippelanschlüsse **59** lassen sich die Leitungen und damit die Elektroden in einen Elektrolytkreislauf integrieren. Es wird zudem vorgeschlagen, die Leitungen in den Bohrungen der Aufnahmen **57** aus- und einmünden zu lassen, wobei die Elektroden mit einer umlaufenden Halterille zwischen zwei ebenfalls umlaufenden Haltebünde zu versehen sind. Der Zu- und Ablauf der Elektroden ist dabei vorzugsweise in der genannten Halterille angeordnet, wobei die Tiefe der umlaufenden Halterille an beiden Seiten zwischen Zu- und Ablauf reduziert ist und somit eine Umleitung des Elektrolytkreislaufstroms durch die Halterille an der Elektrode vorbei behindert wird. Die schematisch dargestellten Leitungen sind optional alternativ oder ergänzend auch als elektrische Messleitungen gestaltbar, wobei in den Aufnahmen zwischen Elektroden und Brillengestelle entsprechende elektrische Schnittstellen vorgesehen sind.

**Fig.9** offenbart eine Ansicht eines Patienten **60** mit einer Videobrille **61** als Elektrodenhalterung mit schematisch dargestellten Aufnahmen **57** mit Elektroden **44** der vorgenannten Art. Die Ausgestaltung sieht ebenfalls vor, die Elektroden vorzugsweise seriell in einem Elektrolytkreislauf einzubinden. Die hierzu erforderliche Umwälzpumpe **46** sowie die gestrichelt dargestellten Leitungen sind vorzugsweise in der Videobrille integriert, wobei die Schnittstelle zwischen Aufnahme und Elektroden vorzugsweise wie anhand **Fig.8** beschrieben gestaltet sind. Optional sind die Leitungen alternativ oder ergänzend auch als elektrische Messleitungen gestaltbar, wobei vorgeschlagen wird, eine vorzugsweise autarke Messdatenerfassung mit Messdatensender und/oder Messdatenspeicher in die Videobrille zu integrieren, beispielsweise wie die dargestellte Umwälzpumpe am Hinterkopf des Patienten.

### Bezugszeichenliste:

1 Gehäuse
2 Gehäusefuß
3 distales Ende eines Gehäusefußes
4 Auflagefläche
5 Hautoberfläche
6 Kanal
7 Kavität
8 Verteilerkanalabschnitt
9 Zulauf
10 Einstich
11 Elektrolytelektrode
12 Deckel
21 Gehäuse
22 Gehäusefuß
23 distales Ende
24 Deckel
25 Elektrolytelektrode
26 Messkabel
27 erste Kavität
28 zweite Kavität
29 erster Kanal
30 zweiter Kanal
31 ringförmige Auflagefläche
32 Umlenkungsvolumen
33 Zulauf
34 Ablauf
35 Messstelle
36 Anschlussnippel
37 Deckeldichtfläche
38Messsginale mit einer Trockenelektrode ohne Ankopplungsmedium
39Messsginale mit einer Trockenelektrode mit Ankopplungsmedium
40Messsginale mit einer in **Fig.2a** **bis d** dargestellten Ausführungsform der Elektrode
41 Stimulationsfrequenz und deren harmonischen Oberfrequenzen des Stimulus
42 Anzahl der Probennahmen N × 10⁴
43 zweiten Markierung
44 Elektrode
45 Schlauchleitung für Elektrolytlösung
46 Umwälzpumpe
47 Hautoberfläche
48 Ultraschallquelle / Vibrationsmotor
49 Einschub für die Elektrolytelektrode
50 Dichtung
51 Wärmeübertragungsstruktur
52 Haltebund/-rille
53 Temperaturmessfühler
54 U-förmiges Ende der Wärmeübertragungsstruktur
55 Einbuchtung für Temperierungsquelle
56 Brille
57 Aufnahmen für Elektroden
58 Leitung
59 Nippelanschluss

## Patentansprüche

1. Elektrode zur nichtinvasiven Messung von elektrischen Körpersignalen auf einer Hautoberfläche **(5),** umfassend
a) ein Gehäuse **(21)** mit einer ersten und einer zweiten Kavität **(27, 28)** für eine Elektrolytlösung im Gehäuse,
b) Mittel für ein Einbringen von Elektrolytlösung die beiden Kavitäten sowie
c) Mindestens eine Elektrolytelektrode **(25)** in zumindest einem der zwei Kavitäten,
wobei
d) am Gehäuse mindestens ein Gehäusefuß **(22)** mit einer ringförmigen Auflagefläche **(31)** für die Hautoberfläche am jeweiligen distalen Ende **(23)** eines jeden Gehäusefu-ßes vorgesehen sind,
e) jeder Gehäusefuß zwei Kanäle **(29, 30),** je ein Kanal vom distalen Ende zur ersten und zweiten Kavität **(28),** aufweist, sowie
f) die beiden Kanäle zum distalen Ende hin offen sind, **dadurch gekennzeichnet, dass**
g) im jedem Gehäusefuß vor dem jeweiligen distalen Ende jeweils von der ringförmigen Auflagefläche umgeben jeweils in ein Umlenkungsvolumen **(32)** zusammengeführt sind.

2. Elektrode nach Anspruch 1, **dadurch gekennzeichnet, dass** am Gehäuse mindestens drei Gehäusefüße **(22)** mit je einer ringförmigen Auflagefläche **(31)** für die Hautoberfläche am jeweiligen distalen Ende **(23)** eines jeden Gehäusefußes vorgesehen sind.

3. Elektrode nach Anspruch 2, **dadurch gekennzeichnet, dass** das die distalen Enden **(23)** und/oder die Auflageflächen **(31)** eine gemeinsame Ebene aufspannen.

4. Elektrode nach Anspruch 2 oder 3, **dadurch gekennzeichnet, dass** alle distalen Enden **(23)** kreisförmig zueinander angeordnet sind.

5. Elektrode nach einem der vorgenannten Ansprüche, **dadurch gekennzeichnet, dass** die das Gehäuse einstückig aus einem druckfähigen Material hergestellt ist.

6. Elektrode nach Anspruch 5, **dadurch gekennzeichnet, dass** das Material ein elektrischer Isolator, vorzugsweise Kunststoff ist.

7. Elektrode nach Anspruch 6, **dadurch gekennzeichnet, dass** das Gehäuse einen Einschub für eine separate Elektrolytelektrode aufweist.

8. Elektrode nach Anspruch 5, **dadurch gekennzeichnet, dass** das Material ein elektrisch leitfähiges Material ist und die Elektrolytelektrode durch Innenwandungen der Kavitäten und/oder der Kanäle gebildet wird.

9. Elektrode nach einem der vorgenannten Ansprüche, **dadurch gekennzeichnet, dass** Mittel zur Temperierung zumindest der Kavitäten vorgesehen sind vorzugsweise umfassend eine Wärmeübertragungsstruktur **(51)** zwischen einer Temperierungsquelle außerhalb des Gehäuses und mindestens einer Kavität sowie ein Temperaturmessfühler **(53)** im Gehäuse.

10. Elektrode nach einem der vorgenannten Ansprüche, **dadurch gekennzeichnet, dass** die Elektrolytelektrode **(25)** eine Elektrode aus Ag, Ag/AgCl, Gold, Platin oder Edelstahl mit oder ohne eine Beschichtung aus Ag/AgCl, Gold, Platin, Edelstahl oder einem anderen leitfähigen Material aufweist.

11. Elektrode nach Anspruch 5, **dadurch gekennzeichnet, dass** die Elektrolytelektrode (25) teilweise oder ganz durch das Gehäusematerial gebildet sind, wobei das Gehäusematerial elektrisch leitfähig ist.

12. Elektrode nach einem der vorgenannten Ansprüche, **dadurch gekennzeichnet, dass** die ringförmigen Auflagefläche **(31)** als Dichtring zu der Hautoberfläche **(5)** gestaltet ist.

13. Elektrode nach einem der vorgenannten Ansprüche, **dadurch gekennzeichnet, dass** das Gehäuse mindestens einen umlaufenden Haltebund und/oder mindestens eine umlaufende Halterille für eine Aufnahme der Elektrode aufweist.

14. Elektrode nach einem der vorgenannten Ansprüche, **dadurch gekennzeichnet, dass** im Gehäuse Ultraschallquellen in mindestens einen der beiden Kavitäten und/oder Kanälen und/oder am Umlenkvolumen angeordnet und in diese ausgerichtet sind.

15. Elektrode nach Anspruch 14, **dadurch gekennzeichnet, dass** die Ultraschallquellen piezokeramische Wandlerelemente jeweils mit einer Auskopplungsschicht umfassen, wobei vorzugsweise die Ultraschallquellen und/oder die Auskopplungsschichten integraler Bestandteil der Gehäusefüße sind und eine fluiddichte elektrisch isolierende Barriere zwischen den Wandlerelementen und den beiden Kavitäten, den Kanälen und dem Umlenkvolumen bilden.

16. Elektrode nach einem der vorgenannten Ansprüche, **dadurch gekennzeichnet, dass** im Gehäuse eine Vibrationsquelle für die Auflagefläche angeordnet ist.

17. Elektrode nach einem der vorgenannten Ansprüche, **dadurch gekennzeichnet, dass** die erste und die zweite Kavität **(27, 28)** sowie die dazwischen angeordneten Kanäle **(29, 30)** in dem Gehäusefuß oder den Gehäusefüßen **(22)** eine Fluidlleitung für die Elektrolytlösung bilden und die Fluidleitung ein Teil eines Elektrolytkreislaufs ist.

18. Elektrode nach einem der vorgenannten Ansprüche, **dadurch gekennzeichnet, dass** ein Zulauf **(33)** für eine Elektrolytlösung zu der ersten Kavität **(27)** im Gehäuse sowie einen Ablauf **(34)** für die Elektrolytlösung aus der zweiten Kavität **(28)** im Gehäuse für eine Elektrolytlösung vorgesehen ist.

19. Elektrode nach Anspruch 18, **dadurch gekennzeichnet, dass** die Elektrolytelektrode **(25)** in der ersten Kavität **(27)** angeordnet ist.

20. Elektrodensystem, umfassend mindestens zwei Elektroden nach Anspruch 18 oder 19, wobei die Elektroden über die Zu und Abläufe in Reihe geschaltet zusammen mit einer Umwälzpumpe **(46)** zu einem Elektrolytkreislauf zusammengeschlossen sind.

21. Elektrodensystem nach Anspruch 20, umfassend weiterhin eine Elektrodenhalterung, vorzugsweise eine Brille **(56)** oder eine Videobrille umfassend, mit Aufnahmen **(57),** vorzugsweise Gummielemente umfassend, der Elektroden.

22. Elektrodensystem nach Anspruch 21, **dadurch gekennzeichnet, dass** die Elektrodenhalterung fluidische Leitungen **(58)** und die Umwälzpumpe **(46)** für den Elektrolytkreislauf und/oder elektrische Leitungen und eine autarke Messdatenerfassung mit Messdatensender und/oder Messdatenspeicher umfasst.

## Claims

1. Electrode for the non-invasive measurement of electric body signals on a skin surface (5), comprising
a) a housing (21) with a first and a second cavity (27, 28) for an electrolyte solution in the housing,
b) means for introducing the electrolyte solution into the two cavities, and
c) at least one electrolyte electrode (25) in at least one of the two cavities,
wherein
d) provided at the housing is at least one housing foot (22) with an annular support surface (31) for the skin surface at the respective distal end (23) of each housing foot,
e) each housing foot has two channels (29, 30), one channel from the distal end to the first and second cavities (28), and
f) the two channels are open towards the distal end,
**characterised in that**
g) the two channels, surrounded by the annular support surface, are combined into a deflection volume (32) in each housing foot, upstream of the respective distal end.

2. Electrode according to claim 1, **characterised in that** at least three housing feet (22), each with an annular-shaped support surface (31) for the skin surface, are provided at the respective distal end (23) of each housing foot.

3. Electrode according to claim 2, **characterised in that** the distal ends (23) and/or the support surfaces (31) span a common plane.

4. Electrode according to claim 2 or 3, **characterised in that** all the distal ends (23) are arranged in a circular form in relation to each other.

5. Electrode according to any one of the preceding claims, **characterised in that** the housing is produced as one piece from a printable material.

6. Electrode according to claim 5, **characterised in that** the material is an electrical isolator, preferably plastic.

7. Electrode according to claim 6, **characterised in that** the housing has an insert for a separate electrolyte electrode.

8. Electrode according to claim 5, **characterised in that** the material is an electrically conductive material and the electrolyte electrode is formed by inner walls of the cavities and/or the channels.

9. Electrode according to any one of the preceding claims, **characterised in that** means are provided for controlling the temperature of at least the cavities, preferably comprising a heat transfer structure (51) between a temperature-controlling source outside the housing and at least one cavity, as well as a temperature sensor (53) in the housing.

10. Electrode according to any one of the preceding claims, **characterised in that** the electrolyte electrode (25) comprises an electrode made of Ag, Ag/AgCI, gold, platinum, or special steel, with or without a coating of Ag/AgCI, gold, platinum, special steel, or another conductive material.

11. Electrode according to claim 5, **characterised in that** the electrolyte electrode (25) is formed in part or in whole by the housing material, wherein the housing material is electrically conductive.

12. Electrode according to any one of the preceding claims, **characterised in that** the annular support surface (31) is configured as a sealing ring to the skin surface (5).

13. Electrode according to any one of the preceding claims, **characterised in that** the housing comprises at least one circumferential holding band and/or at least one circumferential holding groove for receiving the electrode.

14. Electrode according to any one of the preceding claims, **characterised in that** ultrasound sources are arranged in the housing in at least one of the two cavities and/or channels and/or at the deflection volume, and are aligned in them.

15. Electrode according to claim 14, **characterised in that** the ultrasound sources comprise piezoceramic transformer elements, each with a decoupling layer, wherein preferably the ultrasonic sources and/or the decoupling layers are integral parts of the housing feet, and form a fluid-tight electrically isolating barrier between the transformer elements and the two cavities, the channels, and the deflection volume.

16. Electrode according to any one of the preceding claims, **characterised in that** a vibration source for the support surface is arranged in the housing.

17. Electrode according to any one of the preceding claims, **characterised in that** the first and the second cavities (27, 28), and the channels (29, 30) arranged between them, form in the housing foot or the housing feet (22) a fluid line for the electrolyte solution, and the fluid line is a part of an electrolyte circuit.

18. Electrode according to any one of the preceding claims, **characterised in that** an inlet (33) is provided for an electrolyte solution into the first cavity (27) in the housing, and an outlet (34) for the electrolyte solution out of the second cavity (28) in the housing.

19. Electrode according to claim 18, **characterised in that** the electrolyte electrode (25) is arranged in the first cavity (27).

20. Electrode system, comprising at least two electrodes according to claim 18 or 19, wherein the electrodes are connected in series over the inlets and outlets, in order to form, together with a circulating pump (46), an electrolyte circuit.

21. Electrode system according to claim 20, further comprising an electrode holder, preferably a pair of glasses (56) or video glasses, with receivers (57), preferably comprising rubber elements, of the electrodes.

22. Electrode system according to claim 21, **characterised in that** the electrode holder comprises fluidic lines (58), and the circulating pump (46) for the electrolyte circuit and/or electric lines and an independent measured data detector with a measured data transmitter and/or measured data memory store.

## Revendications

1. Electrode de mesure non invasive de signaux électriques corporels sur une surface de peau (5) comprenant :
a) un boîtier (21) avec une première et une seconde cavité (27, 28) pour une solution d'électrolyte dans le boîtier,
b) des moyens pour introduire la solution d'électrolyte dans les deux cavités, et
c) au moins une électrode d'électrolyte (25) dans au moins l'une des deux cavités,
électrode dans laquelle
d) le boîtier a au moins un pied (22) avec une surface d'appui annulaire (31) pour la surface de la peau à chaque extrémité distale (23) de chaque pied de boîtier,
e) chaque pied de boîtier a deux canaux (29, 30), chaque canal allant de l'extrémité distale à la première et la seconde cavité (28), et
f) les deux canaux sont ouverts vers l'extrémité distale,
électrode **caractérisée en ce que**
g) dans chaque pied de boîtier, avant l'extrémité distale respective, entourée par la surface d'appui annulaire, les canaux sont réunis dans un volume de déviation (32).

2. Electrode selon la revendication 1,
**caractérisée en ce que**
le boîtier a au moins trois pieds (22) ayant chacun une surface d'appui (31) annulaire pour la surface de la peau, à l'extrémité distale (23) respective de chaque pied de boîtier.

3. Electrode selon la revendication 2,
**caractérisée en ce que**
les extrémités distales (23) et/ou les surfaces d'appui (31) sous-tendent un plan commun.

4. Electrode selon la revendication 2 ou 3,
**caractérisée en ce que**
toutes les extrémités distales (23) sont réparties les unes par rapport aux autres selon une forme circulaire.

5. Electrode selon l'une des revendications précédentes,
**caractérisée en ce que**
elle est réalisée comme boîtier en une seule pièce en une matière résistant à la pression.

6. Electrode selon la revendication 5,
**caractérisée en ce que**
la matière est un isolant électrique, de préférence une matière plastique.

7. Electrode selon la revendication 6,
**caractérisée en ce que**
le boîtier a un tiroir pour une électrode d'électrolyte distincte.

8. Electrode selon la revendication 5,
**caractérisée en ce que**
la matière est une matière électro-conductrice et l'électrode d'électrolyte est formée par la paroi intérieure des cavités et/ou des canaux.

9. Electrode selon l'une des revendications précédentes,
**caractérisée en ce que**
elle comprend des moyens pour mettre en température au moins les cavités, ces moyens comprenant de préférence une structure thermo-conductrice (51) entre une source de mise en température à l'extérieur du boîtier et au moins une cavité ainsi qu'un capteur de mesure de température (53) dans le boîtier.

10. Electrode selon l'une des revendications précédentes,
**caractérisée en ce que**
l'électrode d'électrolyte (25) est une électrode Ag, Ag/AgCl, Or, Platine, ou acier inoxydable avec ou sans revêtement de Ag/AgCl, Or, Platine, acier inoxydable ou en une autre matière conductrice.

11. Electrode selon la revendication 5,
**caractérisée en ce que**
l'électrode d'électrolyte (25) est formée en partie ou totalement avec le matériau du boîtier, qui est un matériau électro-conducteur.

12. Electrode selon l'une des revendications précédentes,
**caractérisée en ce que**
la surface d'appui annulaire (31) est sous la forme d'un joint annulaire pour la surface de la peau (5).

13. Electrode selon l'une des revendications précédentes,
**caractérisée en ce que**
le boîtier a au moins une collerette de maintien périphérique et/ou au moins une nervure de maintien périphérique pour recevoir l'électrode.

14. Electrode selon l'une des revendications précédentes,
**caractérisée en ce que**
le boîtier comporte des sources d'ultrasons dans au moins l'une des deux cavités et/ou canaux et/ou volume de renvoi et ces sources sont orientées dans celles-ci.

15. Electrode selon la revendication 14,
**caractérisée en ce que**
les sources d'ultrasons comprennent des éléments transducteurs électro-céramique avec une couche de découplage,
de préférence les sources d'ultrasons et/ou les couches de découplage sont des constituants intégrés aux pieds du boîtier et forment une barrière électro-isolante, étanche aux fluides, entre les éléments transducteurs et les deux cavités, les canaux et les volumes de renvoi.

16. Electrode selon l'une des revendications précédentes,
**caractérisée en ce que**
une source de vibrations pour la surface d'appui est dans le boîtier.

17. Electrode selon l'une des revendications précédentes,
**caractérisée en ce que**
la première et la seconde cavité (27, 28) ainsi que les canaux (29, 30) intermédiaires dans le pied du boîtier ou dans les pieds du boîtier (22) forment une conduite de fluide pour la solution d'électrolyte, la conduite d'électrolyte étant une partie du circuit d'électrolyte.

18. Electrode selon l'une des revendications précédentes,
**caractérisée par**
une arrivée (33) de solution d'électrolyte dans la première cavité (27) dans le boîtier ainsi qu'une sortie (34) de la solution d'électrolyte de la seconde cavité (28) dans le boîtier pour la solution d'électrolyte.

19. Electrode selon la revendication 18,
**caractérisée en ce que**
l'électrode d'électrolyte (25) est dans la première cavité (27).

20. Système d'électrodes comprenant au moins deux électrodes selon la revendication 18 ou 19, les électrodes étant branchées en série pour l'entrée et la sortie formant un circuit d'électrolyte avec une pompe de circulation (46).

21. Electrode selon la revendication 20,
comprenant en outre
un support d'électrode comprenant de préférence une lunette (56) ou une lunette vidéo avec des prises (57), de préférence des éléments en caoutchouc pour les électrodes.

22. Electrode selon la revendication 21,
**caractérisée en ce que**
les supports d'électrode sont des conduites fluidiques (58) et la pompe de circulation (46) du circuit d'électrolyte et/ou les lignes électriques et une saisie autonome de données de mesure avec émetteur de données de mesure et/ou mémoire de données de mesure.
